# EUROPEAN PATENT APPLICATION

(11) **EP 4 613 841 A1**
(43) Date of publication of application: **10.09.2025**
(21) Application number: 24161200.1
(22) Date of filing: 04.03.2024
(51) Int. Cl.: C12M 1/32, C12M 3/00, C12M 1/12, C12M 1/42

(54) **MICROWELL FOR CULTURING AN ORGANOID AND DEVICE FOR MEASURING ELECTRICAL ACTIVITY AND/OR STIMULATING CELLS IN A THREE-DIMENSIONAL NETWORK**

(71) Applicant: NMI NATURWISSENSCHAFTLICHES UND MEDIZINISCHES INSTITUT AN DER UNIVERSITÄT TÜBINGEN, 72770 Reutlingen (DE); FEMTOprint SA, 6933 Muzzano (CH)
(72) Inventor: CESARE, Paolo, 39100 Bolzano (IT); VAN DER MOOLEN, Matthijs, 3818 JN Amersfoort (NL); LOVERA, Andrea, 6963 Pregassona (CH); MOMMO, Sacha, 6953 Lugaggia (CH)
(74) Representative: Witte, Weller & Partner Patentanwälte mbB

(57) **Abstract**

The present invention relates to a microwell for culturing an organoid at a distance from the wall of the microwell and allowing development of a three-dimensional network, and to a device for measuring electrical activity of and/or stimulating cells in a three-dimensional network.

## Description

The present invention relates to a microwell for culturing an organoid at a distance from the wall of the microwell and allowing development of a three-dimensional network, and to a device for measuring electrical activity of and/or stimulating cells in a three-dimensional network.

### FIELD OF THE INVENTION

The present invention relates to the field of biomedical research, especially to cell culture, more specifically to culturing an organoid in a three-dimensional network, and further specifically to investigating behavior of an organoid in a three-dimensional network.

### BACKGROUND

Cell culture allows to maintain cells derived from an organism alive in a controlled environment. Classically, this is done for research purposes, but also for purposes of cell therapy. To maintain and observe the physiological behavior of the cells, the environment of the cells is to be controlled such that it resembles as far as possible the physiological environment of the cells.

In multicellular organisms, each cell is in a physiological three-dimensional environment, allowing and promoting physiological behavior of the cell. To investigate the behavior of cells within the physiological three-dimensional environment is of interest in the field since in the past years, techniques, especially visualization techniques, to investigate single cells at a cellular or even molecular level have developed and gained much interest. This especially applies to neuronal cells, which represent highly interactive cells that develop complex, multicellular, three-dimensional networks.

To the extent those techniques reveal details invisible before, they are limited by observation of artifacts caused by alterations in the environment of the cells. Investigation in a physiological three-dimensional environment can be performed by techniques to culture organs. However, those are still limited in that within organs, the cells, especially single cells, are difficult to be kept alive and accessed.

Therefore, in the past years, techniques to culture groups of cells, so called organoids, have been developed. Often, culture starts with progenitor cells of the desired cell type, which progenitor cells are differentiated in vitro into the desired cell type by addition of defined combinations of growth factors.

Prior art methods to culture an organoid comprise injection of cells into a hydrogel such that a bubble is formed inside the gel, in which the cells are compressed to some extent and, thus, aggregate to form an organoid. However, such methods do not allow reliable reproducibility of the positioning of the organoid and result in limited accessibility.

Other prior art methods, as for example the patch-clamp-technology measure and stimulate electrical activity of single neuronal cells. Additionally, in the past years multi-electrode-arrays have been developed for measuring and stimulating groups of neuronal cells. EP 3 494 877 A1 discloses a device for measuring and stimulating groups of neurons in a three-dimensional network. However, the group of neurons, which can be an organoid, is cultured in a cell seeding area at the bottom of a reservoir, where it does not have a fixed position.

Thus, reproducible assessment, for example visually, and accessibility, for example for manipulation, and efficiency of three-dimensional extension of the organoid cultured in a three-dimensional network is impaired.

Thus, it is an object of the present invention to provide a device allowing easy and reproducible positioning of an organoid for three-dimensional culture.

### SUMMARY OF THE INVENTION

In an aspect of the invention, the above identified disadvantages and others are overcome by providing a microwell for culturing an organoid, the microwell comprising a main body, a sample bed, and at least one positioning member,
the main body comprising a wall and a bottom,
the at least one positioning member comprising
a first end, and
a second end attached to the main body,
wherein the sample bed is formed at least partially by the first end.

The inventors found that defined, fixed positioning of an organoid for three-dimensional culture solves the object. The microwell according to the invention advantageously allows to target the organoid repeatedly in a standardized manner, for example by machines configured for manipulation, measuring and/or visualization. Additionally, such fixed positioning improves reproducibility of experimental data when repeating an experiment as well as comparability when performing two or more experiments in parallel. Moreover, by use of the microwell, the organoid is kept at a distance from the wall and the bottom of the microwell, which optimizes three-dimensional culture since the organoid is minimally impaired to extend.

The term "microwell" refers to a vessel holding a volume of liquid of no more than 20 mL, preferably no more than 10 mL, further preferably no more than 1 mL, most preferably no more than 500 µL. Preferably, the vessel has a single opening for filling and/or emptying the vessel. However, the vessel may have further openings.

The microwell can be a flat-bottom microwell or a curved-bottom microwell, preferably a flat-bottom microwell.

The term "organoid" is to be understood as an aggregated group of cells, which cells have been derived or differentiated from progenitor cells, for example pluripotent or multipotent stem cells and/or from primary cells, i.e., cells isolated from a living organism, as to resemble or mimic an organ or a group of organs. Typically, such organoid is substantially spherical in shape. The organoid contains one or more types of adherent cells and may comprise non-adherent cells. Preferably, the organoid is substantially spherical. Typical sizes of organoids range from 0.2 mm to 1 mm.

The organoid to be inserted into the microwell may have any size. Preferably, the organoid has a diameter of 0.2 mm to 0.8 mm, more preferably of 0.4 mm to 0.6 mm, most preferably of 0.5 mm.

The main body comprises a wall and a bottom. The surface of the bottom lining the inner volume of the microwell may be flat or concave, preferably flat.

The "sample bed" is the place, where the organoid is to be inserted. In the sample bed, the organoid is fixedly positioned. "Fixedly positioned" is to be understood such that the organoid does not roll or slide around within or out of the sample bed, when the microwell is handled or stands still. However, the organoid is, as the skilled person recognizes, not fixedly attached to or in the sample bed, i.e., the organoid may be removed from the sample bed or may be forced out of the sample bed, for example, when the microwell is shaken or intensively moved.

The sample bed is formed at least partially by the first end. Thus, the sample bed may be additionally formed partially by other means. To at least partially form the sample bed, the first end can be a lateral boundary or lateral boundary and bottom boundary of the sample bed. The sample bed is configured to position the organoid fixedly at least in a plane, i.e., at least in two dimensions. This is achieved by the sample bed being formed at least partially by the first end and the first end being configured as to contact the organoid when the organoid is inserted.

In a preferred embodiment, the sample bed is configured to position the organoid in a plane, i.e., in two dimensions. In this embodiment, the first end of the at least one positioning member forms at least a part of a lateral boundary of the sample bed.

In this embodiment, a bottom of the sample bed can be formed at least partially by a hydrogel, filled into the microwell up to a height immediately underneath the first end. Thus, the sample bed can be formed by the first end of the at least one positioning member together with the hydrogel. This advantageously positions the organoid easily, fixedly, and reproducibly and keeps it easily accessible.

In another preferred embodiment, the sample bed is configured to position the organoid in a three-dimensional space, i.e., to hold the organoid within the inner volume of the microwell. In this embodiment, the first end of the at least one positioning member forms at least a part of a lateral boundary and a part of a bottom boundary of the sample bed. Furthermore, this allows to culture an organoid either within a hydrogel or within a liquid culture medium or partially in a hydrogel and within a liquid culture medium.

In this embodiment, the first end preferably has a pyramid, cone, frustopyramid, frustocone, or a convex shape. Such shape of the first end advantageously allows the first end to extend at least partially at the lateral boundary and at least partially underneath the organoid. Thus, the supporting member advantageously positions the organoid not only in a plane, i.e. in two dimensions, but in space, i.e., in three dimensions.

In this embodiment, the microwell can be filled with a hydrogel up to a height underneath, immediately underneath, equal to, or above the first end.

To have the microwell filled with hydrogel up to a height equal to or above the first end, is advantageous since this way the whole organoid is inside the hydrogel and may grow and extend within all directions.

To have the microwell filled with hydrogel up to a height immediately underneath the first end, is advantageous since this way the organoid is highly accessible but still can efficiently grow three-dimensionally into multiple directions into and within the hydrogel.

In another embodiment, the first end of at least one positioning member is a free end not connected to other first ends and/or the main body other than via the second end.

Preferably, the first end of each positioning member is a free end not connected to other first ends and/or the main body other than via the second end.

In another embodiment, the microwell comprises a single positioning member and the first end comprises a ring configured for holding an organoid.

In this embodiment, the first end at least partially forms the sample bed by comprising the ring. The ring advantageously allows to place an organoid on top of it such that the organoid is hold and fixedly positioned by the ring. Preferably, the ring contacts a bottom hemisphere of a substantially spherical organoid when such organoid is inserted into the microwell.

In this embodiment, the first end is a free end.

The ring may be flat or bent and may have any cyclic shape. Preferably the ring is substantially flat and oriented substantially horizontally, more preferably flat and oriented horizontally. In embodiments, wherein the ring is flat, the ring may have, for example, the shape of a circle, oval, square, or another polygonal shape. Preferably the ring has the shape of a circle or an oval, more preferably the ring has the shape of a circle.

The ring can comprise a gap. The gap can represent from 1% to 50%, preferably from 10% to 30% of the perimeter of the ring. A ring comprising a gap can be, for example U-shaped.

In preferred embodiments, in which the ring has the shape of a circle, the ring has an inner diameter of 0.2 mm to 3 mm, preferably of 0.3 mm to 2 mm, most preferably of 0.5 mm to 1 mm. Such dimensions of the ring advantageously correlate to typical dimensions of organoids.

In a preferred embodiment, the ring is a closed ring.

In another embodiment, the microwell comprises at least 2 positioning members, preferably at least 3, more preferably at least 4, even more preferably at least 5, most preferably 5. Preferably, the microwell does not comprise more than 10 positioning members.

The microwell comprising at least 2 positioning members allows to place an organoid in between the first ends of the at least 2 positioning members. In an embodiment, comprising 2 positioning members, at least one of the positioning members can be, for example Y-shaped, which advantageously achieves a positioning of the organoid at least in a plane.

The microwell comprising at least 3 positioning members allows to place an organoid in between the first ends of the at least 3 positioning members, which advantageously achieves a positioning of the organoid at least in a plane.

The less positioning members are provided, the less is the inner volume of the microwell occupied, which advantageously improves visibility of the content of the microwell, especially the organoid, and accessibility of the inner volume of the microwell. Furthermore, a free inner volume of the microwell improves the capabilities of the organoid to grow and extend equally, in a three-dimensional manner into all directions.

The more positioning members are provided, the safer the organoid will be positioned. However, the freedom of the organoid to grow and extend will be limited. Therefore, preferably no more than 10 positioning members are provided.

In another preferred embodiment, each of the at least one positioning member extends in a plane.

Preferably the plane is horizontal.

In one embodiment, the at least one positioning member is a filled triangle, wherein one angle of the triangle is the first end, and the opposite leg of the triangle is the second end. Preferably the triangle is an isosceles triangle, or an equilateral triangle. More preferably, the triangle is an acute isosceles triangle, wherein the acute angle is the first end, and the opposite leg is the second end. Preferably, the angle being the first end is flattened or rounded, i.e., the triangle is not a perfect triangle.

In a preferred embodiment, the at least one positioning member is a strut, which strut can be straight, bent, bifurcated, or angled. Preferably, the at least one positioning member is a straight or bifurcated strut, more preferably an I-shaped, Y-shaped or T-shaped strut.

The at least one positioning member being a strut advantageously reduces a barrier-effect of the positioning member within the inner volume of the microwell, to allow free growth and extension of the organoid.

A positioning member can be Y-shaped. The base of the Y can be the first end, and the two tip-extensions of the Y can be the second end or vice versa, i.e., the two tip extensions of the Y can be the first end, and the base of the Y can be the second end. A Y-shaped positioning member advantageously allows to reduce the number of positioning members by allowing to contact an organoid at two points. In embodiments, wherein the at least one positioning member is Y-shaped, at least two positioning members need to be provided.

A positioning member can be V-shaped, wherein the base of the V is the first end, and the two tip-extensions of the V are the second end. A V-shaped positioning member advantageously improves accessibility of the inner volume of the microwell at its periphery.

A positioning member can be T-shaped, wherein the horizontal part of the T is the first end, and the base of the T is the second end. A T-shape of a positioning member advantageously enlarges a contact-surface between the first end and an organoid, which improves its positioning.

A positioning member can be a straight strut, which means it is I-shaped. An I-shaped positioning member advantageously reduces the barrier-effect of the positioning-member to a minimum. In embodiments, wherein the at least one positioning member is T-shaped or I-shaped, at least three positioning members need to be provided.

In a preferred embodiment, all positioning members are identically shaped.

In a preferred embodiment, the sample bed and all positioning members extend in the same plane. This advantageously reduces the occupation of the inner volume of the microwell to a single plane, such that the remaining three-dimensional volume of the microwell can be free and available for growth and extension of the organoid. Additionally, this improves visibility of the organoid at least via the bottom of the microwell.

In another preferred embodiment, in which the microwell comprises at least 2 positioning members, the first end of the at least two positioning members comprises a fraction of a ring, which fraction of a ring is connected to at least one other fraction of a ring such that a ring configured for holding an organoid is built. Advantageously, the ring is fixated to the microwell via two positioning members, which improves its stability.

In another preferred embodiment, the microwell comprises a first compartment comprising the sample bed and configured to contain a hydrogel.

The term "compartment" is to be understood hereinafter as a structurally demarcated volume. The volume is not closed, i.e., it is in fluid communication with an external volume, which can be another compartment. As will be clear to the skilled person, a microwell by definition comprises a compartment, which compartment is the inner volume of the microwell. To this inherent compartment of a microwell, it is referred herein as "inner volume". However, the microwell may comprise structural elements demarcating different regions, i.e., compartments of the inner volume.

The first compartment comprising the sample bed and configured to contain a hydrogel, advantageously allows to culture an organoid inserted into the sample bed within or on top of a hydrogel.

Additionally, the first compartment can comprise at least a part of at least one of the at least one positioning member. Preferably, the first compartment comprises all positioning members.

The first compartment can hold a volume up to the volume hold by the microwell. In a preferred embodiment, the first compartment holds a volume of 2 µL to 150 µL, preferably of 10 µL to 50 µL.

In a preferred embodiment, the microwell comprises a second compartment in fluid communication with the first compartment and configured to contain a liquid culture medium, wherein the second compartment is located above the first compartment with respect to gravity.

The second compartment can be structurally demarcated from the first compartment by the sum of the positioning members or by other structural means, as for example the inner shape of the microwell. Preferably, the second compartment holds a larger volume than the first compartment.

The second compartment is in fluid communication with the first compartment and configured to contain a liquid culture medium, wherein the second compartment is located above the first compartment with respect to gravity. This advantageously allows to first fill the first compartment with a hydrogel, let the hydrogel solidify after or prior to inserting the organoid into the sample bed, and to afterwards fill the second compartment with liquid culture medium. Advantageously, this prohibits dilution of the hydrogel prior to its solidification by liquid culture medium and allows supplements from the liquid culture medium to diffuse into the hydrogel.

In a preferred embodiment, the second compartment is configured to contain the liquid culture medium such that the first compartment is fully covered by the liquid culture medium when the liquid culture medium is contained in the second compartment. This advantageously protects the hydrogel from drying out.

The microwell can comprise more than two compartments, preferably up to 3.

In another preferred embodiment, the organoid comprises neuronal cells. The microwell is advantageously used for culture of an organoid comprising neuronal cells. Neuronal cells usually develop and/or comprise a plurality of elongated extensions, so called dendrites and axons. At the dendrites and/or axons, neuronal cells may build connection to other cells, usually neuronal cells and/or muscle cells, which connections are called synapses. Thus, the behavior of the neuronal cells can be better observed with an optimized three-dimensional culture.

In another preferred embodiment, the microwell is made at least partially from glass or plastic. Preferably the microwell is made substantially from glass or plastic, i.e., to at least 90% of its mass, most preferably the microwell is made exclusively from glass or plastic.

The microwell being made substantially or exclusively from glass, advantageously improves thermal resistance of the microwell, such that the microwell is autoclavable.

The microwell being made substantially or exclusively from plastic, advantageously allows fabrication of the microwell by standard microwell-production methods of prior art, which are well known to the skilled person.

In a preferred embodiment, the microwell is made from a material selected from the group consisting of: fused silica, borosilicate, allumosilicate, titania-silicate, soda-lime glass, and an elastomer, more preferably from fused silica, borosilicate, allumosilicate, titania-silicate, and soda-lime glass. Most preferably the microwell is made from fused silica.

Preferably, the material the microwell is made from is autoclavable. This advantageously allows to sterilize and/or reuse the microwell.

Fused silica, borosilicate, allumosilicate, titania-silicate, and soda-lime glass are advantageous due to their chemical inertness, which prohibits unwanted reactions between the material and any contents of the microwell, as for example the liquid culture medium and/or any supplements. Fused silica is particularly advantageous for use of the microwell in combination with an imaging device. This is due to its low autofluorescence, improving use in combination with, for example, a fluorescent microscope, due to its optical transparency in the UV-range, improving use in combination with, for example, a UV-microscope, and due to its low thermal expansion, improving use in combination with precision measuring instruments.

In another preferred embodiment, the microwell is at least partially transparent. This advantageously improves optical or visual accessibility of the organoid. Preferably, at least a part of the bottom is transparent, more preferably, the whole microwell is transparent.

In another preferred embodiment, the microwell is for use in combination with an imaging-device, preferably a microscope. This advantageously allows imaging of the organoid during culture and/or experimentation.

In another aspect, the object is solved by providing a device for measuring electrical activity of and/or stimulating cells in a three-dimensional network, the device comprising at least one microwell according to the invention.

The term "three-dimensional network" refers to the spatial connection of a plurality of cells in a biocompatible matrix allowing the cultivation of the cells. Preferably, the cells interact electrically and preferably the plurality of cells includes neuronal cells.

The device comprising at least one microwell according to the invention advantageously allows to measure electrical activity and/or electrically stimulate an organoid cultured in a three-dimensional network with the organoid being positioned fixedly and reproducibly and kept at a distance from the wall and the bottom of the microwell.

In a preferred embodiment, the microwell is made substantially or exclusively from glass.

In a preferred embodiment, the organoid comprises neuronal cells. Neuronal cells are advantageously known to show electrical activity, to be electrically stimulable and to develop a three-dimensional network.

In a preferred embodiment, the device comprises from up to 5000 microwells, preferably up to 3456, more preferably up to 3456, 1536, 384, 96, 48, 24, 12, 11, 10, 9, 8, 7, 6, 5, 4, 3, or 2 microwells, even more preferably 96, 10, 9, 8, 7, 6, 5, 4, 3, or 2 microwells, most preferably 9 microwells. This advantageously allows to conduct a plurality of experiments in parallel and facilitates handling of the respective associated number of microwells, especially with respect to high-throughput experiments.

In a preferred embodiment comprising more than one microwell, the microwells are distributed in a regular grid.

In another preferred embodiment, the bottom of the at least one microwell comprises a microelectrode array (MEA) comprising at least one microelectrode.

The term "microelectrode" refers to any device capable of measuring electrical activity of and/or stimulating, preferably electrically, cells. A microelectrode has suitable properties to its function, including low impedance and low noise. The microelectrode may be made of a suitable material for performing its function, including but not limited to gold, platinum, iridium, iridium oxide, titanium nitride, carbon nanotubes, graphene, diamond, a conducting polymer such as poly(3,4-ethylene-dioxythiophene) or combinations of these materials. The microelectrode may include a coating which enables or improves its function.

It is to be understood that the same microelectrode can be arranged to measure electrical signals from neurites and/or be arranged to administer electrical signals to neurites. I.e., the at least one microelectrode can have both or only one of the mentioned functions.

In another preferred embodiment, the bottom of the at least one microwell comprises at least one microchannel, comprising a wall, being in fluid communication with the first compartment, and being dimensioned as to allow extension of neurites from the first compartment into said microchannel and prevent the entry of the soma of neurons, and wherein an inner wall of the microchannel is formed at least partially by at least one microelectrode.

In a preferred embodiment, the bottom of the at least one microwell comprises up to 1000 microchannels, preferably up to 500, and most preferably up to 100. This advantageously allows to electrically measure and/or stimulate a plurality of cells of a three-dimensional network.

In a preferred embodiment, the at least one microchannel comprises a height and/or width of 2 µm to 20 µm, further preferably of 3 µm to 10 µm. Such dimensions advantageously allow extension of neurites from the first compartment into the at least one microchannel, while preventing the entry of the soma of neurons. The length of a microchannel can be from 50 µm to 500 µm, preferably from 200 µm to 300 µm.

The at least one microchannel is in fluid communication with the first compartment. This means, the at least one microchannel opens into the first compartment.

Preferably, the at least one microchannel is in fluid communication with the first compartment via at least one entrance. Thus, the at least one microchannel can be a pass-through microchannel, a dead-end microchannel or a pass-through microchannel comprising a dead end. As will be clear to the skilled person, such entrance can also serve as an exit, without any structural adaptations.

The at least one microchannel can be straight, twisted, branched, curved, bent, or a combination thereof.

The wall of the microchannel is formed at least partially by at least one microelectrode. The wall advantageously enhances the electrical isolation of the at least one microelectrode, which reduces background signals and, thus, amplifies true signals to be measured by a microelectrode.

The microelectrode may be positioned at any position along the length or perimeter of the microchannel. Further, there may be more than one microelectrode per microchannel. The microelectrode may be positioned on one side of the microchannel, along the whole perimeter of the microchannel, may be smaller than the length of the microchannel, or it may extend over the entire length of a microchannel.

In another preferred embodiment, the microchannel is branched. This advantageously allows to measure electrical activity and/or stimulate cells that were forced to grow angled.

In another preferred embodiment, the microchannel comprises at least one Y-shaped segment, preferably at least 2, further preferably at least 4.

In another preferred embodiment, preferably at least two Y-shaped segments, further preferably 4, are connected at the Y's base.

In another preferred embodiment, the at least one microelectrode and the at least one microchannel is distributed within a ring-shaped region of the bottom of the first compartment and the sample bed is positioned vertically above the center of the ring-shaped region. This advantageously allows the organoid to extend equally through all microchannels since the organoid usually will extend radially from the sample bed.

In this embodiment, preferably at least one of the at least one microchannel is a pass-through microchannel.

In another preferred embodiment, the device is for use in combination with an imaging-device, preferably a microscope. This advantageously allows imaging of the organoid during culture and/or electrical measuring or stimulation.

In another preferred embodiment, the device comprises a handling member. The handling member can be an extension or overhang of the device extending laterally or to the top and configured to be grasped by a user when handling the device. Such handling member advantageously facilitates handling of the device.

In another preferred embodiment, visual guides are provided, which visual guides mark or guide to the sample bed. Preferably, the visual guides are provided on at least one of the at least one positioning member and/or on the bottom of the microwell, more preferably, on top of at least one of the at least one positioning member or on the bottom of the microwell at a position located vertically under the sample bed.

It is understood that the features mentioned above and those yet to be explained below can be used not only in the particular combination given, but also in other combinations or in isolation, without departing from the scope of the present invention. Especially, the features, characteristics, embodiments, and advantages mentioned with respect to the bioreactor similarly apply to the system comprising such bioreactor.

The invention is now described and explained in further detail by referring to the following non-limiting examples and drawings.

### BRIEF DESCRIPTION OF THE DRAWINGS

Fig. 1A shows a sketch of the microwell for culturing an organoid as a top-view.
Fig. 1B shows a sketch of the microwell for culturing an organoid as a sectional view onto the plane A indicated in Fig. 1A.
Fig. 1C shows the microwell comprising a single positioning member as a top-view.
Fig. 1D shows the microwell comprising 2 positioning members as a top-view.
Fig. 1E shows the microwell comprising 2 positioning members connected to one or more first ends of other positioning members as a top-view.
Fig. 2A shows the microwell comprising 2 compartments and 5 positioning members as a top-view.
Fig. 2B shows the microwell comprising 2 compartments as a section-view onto the plane II indicated in Fig. 2A.
Fig. 3 shows a positioning member of a microwell as a top-view.
Fig. 4 shows a positioning member of a microwell as a side-view.
Fig. 5 shows a sample bed with an organoid inserted.
Fig. 6A shows the device for measuring electrical activity of and/or stimulating cells in a three-dimensional network as a three-dimensional view.
Fig. 6B shows the device for measuring electrical activity of and/or stimulating cells in a three-dimensional network as a top-view.
Fig. 7 shows a vertical section through the bottom of the microwell.
Fig. 8 shows a horizontal section through the bottom of the microwell with examples of microchannels.
Fig. 9 shows a sketch of the device, with an organoid inserted.
Fig. 10 shows a microscopic photo of a sample bed of the device, with an organoid inserted.
Fig. 11 shows is a microscopic photo of a microchannel distributed within a region of the bottom.
Fig. 12 shows a measurement obtained at 4 days after plating an organoid comprising neuronal cells in the device comprising the microwell.

### DETAILED DESCRIPTION OF PREFERRED EMBODIMENTS

### 1. Microwell

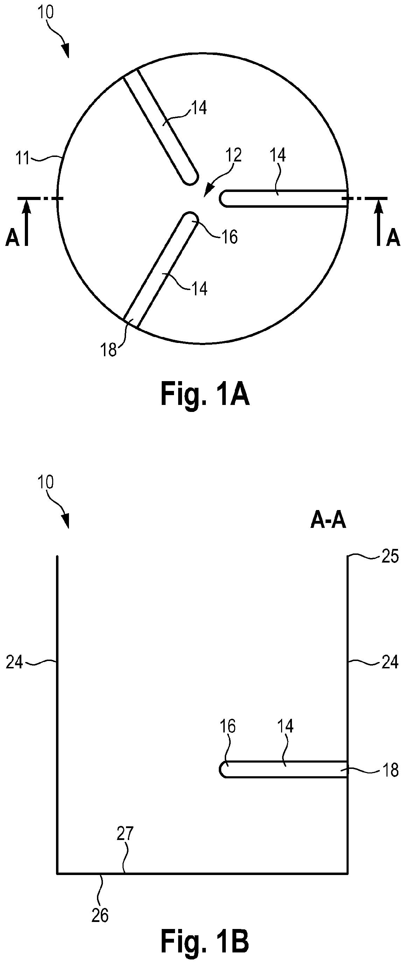

Fig. 1A shows a sketch of a microwell 10 for culturing an organoid as a top-view. Fig. 1B shows sketch of a vertical section through the microwell 10. The microwell 10 comprises a main body 11, a sample bed 12, and three positioning members 14.

The main body 11 comprises a wall 24 and a bottom 26.

The wall 24 comprises an upper rim 25, extends upwards from the bottom 26, such that the bottom 26 and the wall 24 together hold a compartment or inner volume, i.e., confer to the microwell 10 the basic characteristics of a vessel.

The inner volume of the microwell 10 can be of any shape. Preferably it is overall cylindrical. The inner volume is no more than 20 mL, preferably no more than 10 mL, further preferably no more than 1 mL, most preferably no more than 500 µL.

The upper rim 25 forms an opening, which can serve as an inlet into and/or outlet from the microwell 10. In other preferred embodiments the opening can be configured to be closed by a lid (not shown). The lid can be any type of closure, as for example a stuck foil, a screwed lid, or a plugged lid.

The bottom 26 comprises a first surface 27 lining the inner volume. The first surface 27 can be flat. In other preferred embodiments, the first surface 27 can be concavely curved.

The wall 24 and the bottom 26 can be attached to each other in a fluid-tight manner. An attachment can be achieved by any means, as for example gluing, sticking, or screwing. Attachment is preferably achieved by fabricating the wall 24 and the bottom 26 from one piece.

Each positioning member 14 comprises a first end 16 and a second end 18.

In the embodiment of microwell 10, three positioning members 14 are provided. In other preferred embodiments, at least one positioning member 14 can be provided. Preferably 1, 2, 3, 4, 5, 6, 7, 8, 9, or 10 positioning members are provided, more preferably, 1, 4, 5, or 6, and most preferably 5.

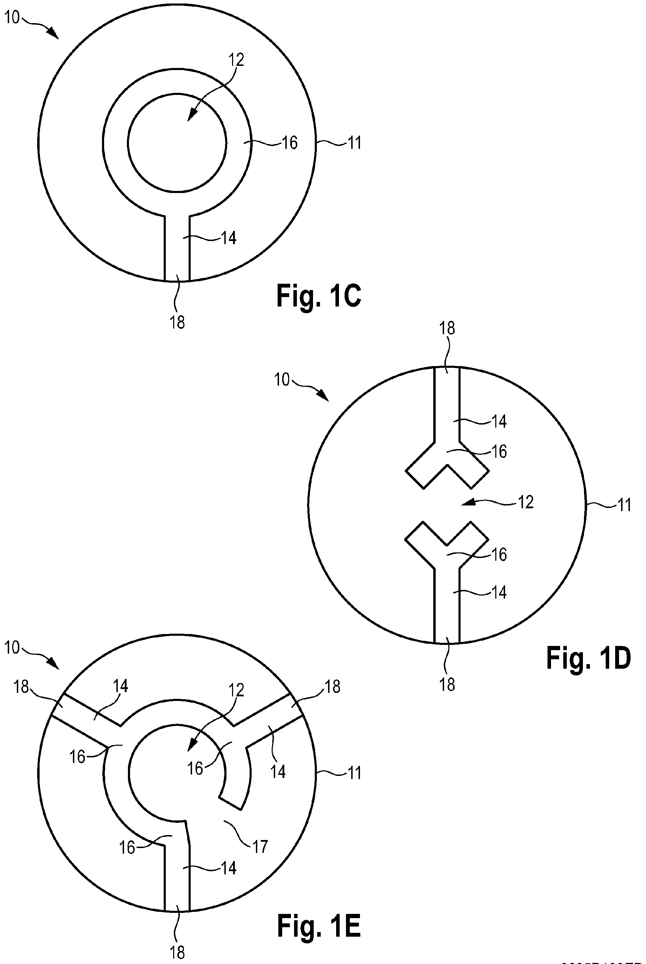

Fig. 1C shows an embodiment of microwell 10 comprising a single positioning member 14 as a top-view. The first end 16 may comprise a ring. In other preferred embodiments, the ring may comprise a gap 17 (see Fig. 1E). The gap 17 can represent from 1 % to 50%, preferably from 10% to 30% of the perimeter of the ring.

Fig. 1D shows an embodiment of microwell 10 comprising 2 positioning members 14 as a top-view. The positioning members 14 can be bifurcated or Y-shaped.

The first end 16 can be a free end, wherein the first end 16 is not connected to the main body 11 other than via the second end 18, as shown in Fig. 1A, 1C, or 1D. In other embodiments, the first end 16 can be connected to one or more first ends 16 of other positioning members 14. The first end 16 can be configured to contact the organoid, when the organoid is inserted.

Fig. 1E shows an embodiment of microwell 10 comprising 3 positioning members connected to one or more first ends 16 of other positioning members 14 as a top-view. The first ends 16 can comprise each a fraction of a ring, which fraction of a ring is connected to at least one other fraction of a ring such that a ring configured for holding an organoid is built. The ring can comprise a gap 17. The gap 17 can represent from 1% to 50%, preferably from 10% to 30% of the perimeter of the ring. In other preferred embodiments, the ring may not comprise a gap 17 (see Fig. 1C).

In all embodiments shown in Fig. 1, the at least one positioning member 14 can extend in a plane and all positioning members 14 and the sample bed 12 can extend in the same plane. In other preferred embodiments, at least one of the at least one positioning member 14 may not extend in a plane and/or at least one positioning member 14 can extend outwards a plane in which another positioning member 14 extends.

The second end 18 is attached to the main body 11, preferably to the wall 24. The attachment can be achieved by any means, as for example gluing, sticking, or screwing. Attachment is preferably achieved by fabricating the main body 11 and the positioning member 14 from one piece.

In other preferred embodiments, the second end 18 can be attached to the bottom 26.

The sample bed 12 is at least partially formed by the first end 16 of the at least one positioning member 14 and is the place, where the organoid is to be inserted. To at least partially form the sample bed 12, the first end 16 can be a lateral boundary for the sample bed 12. In other preferred embodiments, the first end 16 can be a lateral boundary and at the same time a bottom boundary of the sample bed 12.

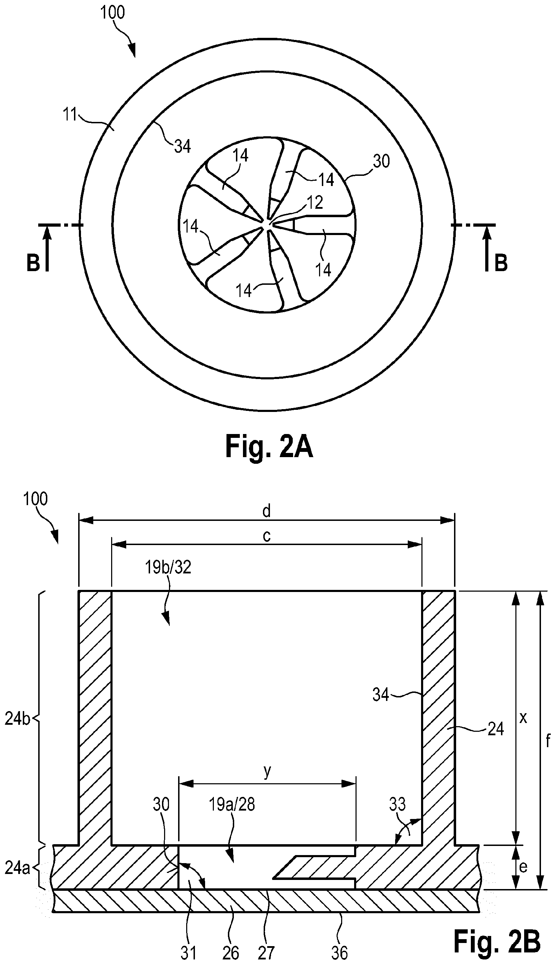

Fig. 2A shows a microwell 100, being a preferred embodiment, as a top-view and Fig. 2B shows microwell 100 as a section-view onto the plane B indicated in Fig. 2A. Features of microwell 100 similar or identical to the features described with respect to microwell 10 are labeled by the same reference signs. The microwell 100 comprises a main body 11, a sample bed 12, 5 positioning members 14, a first compartment 19a, and a second compartment 19b.

The wall 24 comprises a first section 24a, and a second section 24b and has a height f of 6.8 mm.

In other preferred embodiments, the height f can be from 3.5 mm to 16 cm, preferably from 5.5 mm to 2.2 cm, more preferably from 5.8 mm to 9.2 mm.

The first section 24a can be a plate comprising a first through-hole 28 and having a height e of 1 mm. In other preferred embodiments, the first section 24a can taper towards the first through-hole 28.

In other preferred embodiments, the height e can be from 0.5 mm to 10 mm, preferably 0.5 mm to 2 mm, more preferably from 0.8 mm to 1.2 mm.

The first through-hole 28 can be circular and comprise a first diameter y of 4 mm and a first inner surface 30.

In other preferred embodiments, the first diameter y can be from 2 mm to 30 mm, preferably from 4 mm to 8 mm, more preferably from 4 mm to 6 mm.

In other preferred embodiments, the first through-hole 28 can have any shape, preferably a cyclic and/or regular shape.

The first inner surface 30 can extend upwards from and in a first angle 31 of 90° relative to the bottom 26 and includes the first compartment 19a.

In other preferred embodiments, the first inner surface 30 can extend upwards and, in a first angle 31 of 20° to 179.99° relative to the bottom 26. Preferably, the first angle 31 is from 90° to 179.99°, more preferably from 90° to 120°.

The second section 24b can be a block, can comprise a second through-hole 32 and can have a height x of 5.8 mm. The second section 24b is attached to the first section 24a such that the first through-hole 28 opens into the second through-hole 32. Thus, the first compartment 19a and the second compartment 19b are in fluid-communication.

In other preferred embodiments, the height x can be from 1 mm to 15 cm, preferably from 5 mm to 2 cm, more preferably from 5 mm to 8 mm.

The second section 24b can be cylindrical and comprise an outer diameter d of 8.5 mm.

In other preferred embodiments the outer diameter d can be from 5 mm to 50 mm, preferably from 7 mm to 12 mm, more preferably from 8 mm to 10 mm.

In other preferred embodiments, the second section 24b can comprise any outer shape, for example a cuboid shape.

The second section 24b can be attached to the first section 24a by any means, as for example gluing, sticking, or screwing. Attachment is preferably achieved by fabricating the first section 24a and the second section 24b from one piece.

The second through-hole 32 can be circular and comprise a second diameter c of 7 mm, and a second inner surface 34.

In other preferred embodiments, the second diameter c can be from 3 mm to 48 mm, preferably from 5 mm to 10 mm, more preferably from 6 mm to 8 mm. The second diameter can be larger, identical, or smaller than the first diameter y. Preferably, the second diameter c is larger than the first diameter y.

In another preferred embodiment, the outer diameter d is chosen as to be up to 4 mm, preferably from 0.8 mm to 2 mm larger than the second diameter c.

The first through-hole 28 and the second through-hole 32 can be aligned as to share a common center axis (not shown). In other preferred embodiments, the center axis of the first through-hole 28 and the second through-hole 32 can be different from each other, however, they are preferably parallel to each other, more preferably identical.

The second inner surface 34 can extend upwards from the first portion 24a and in a second angle 33 of 90° relative to the bottom 26 and includes the second compartment 19b.

In other preferred embodiments, the second angle 33 may be from 20° to 179.99° relative to the bottom 26. Preferably, the angle 33 is from 90° to 179.99°, more preferably from 90° to 120°. In another preferred embodiment, the first angle 31 and the second angle 33 are identical.

The bottom 26 can be a base plate and can comprise a first surface 27 lining the inner volume of microwell 100 and a second surface 36. The bottom 26 can have any shape and height and can cover the first through-hole 28. The bottom 26 is attached to the first section 24a.

The first surface 27 lines the first compartment 19a, i.e., the inner volume of microwell 100, and can be flat. In other preferred embodiments, the first surface 27 can be concave.

The second surface 36 is configured to contact a working-place, for example a laboratory bank or any type of rack or holding-device. In other preferred embodiments, at the second surface 36, there can be provided protrusions (not shown) serving as feet.

In a preferred embodiment, bottom 26 closes the first through-hole 28 fluid-tight. In other preferred embodiments, within the wall 24 or the bottom 26 or in between the bottom 26 and the first section 24a, there may be provided fluid-permeable connections to further compartments.

The microwell 100 can comprise a first compartment 19a comprising the sample bed 12 and configured to contain a hydrogel, and a second compartment 19b in fluid communication with the first compartment 19a and configured to contain a liquid culture medium, wherein the second compartment 19b is located above the first compartment 19a with respect to gravity.

In other preferred embodiments, the first compartment 19a and the second compartment 19b each can contain parts of the sample bed 12. In such embodiments, the at least one positioning member 14 may extend from one compartment into another.

In other preferred embodiments, the microwell 100 can comprise a single compartment (see Fig. 1) or more than two compartments, preferably up to 3.

In other preferred embodiments, as for example shown in Fig. 1, a single compartment can be configured to contain both a hydrogel and a liquid culture medium.

In other preferred embodiments, the second compartment 19b can be located at least partially above the first compartment 19a with respect to gravity. In all embodiments, the hydrogel is preferably covered completely by liquid culture medium and the microwell, when in use.

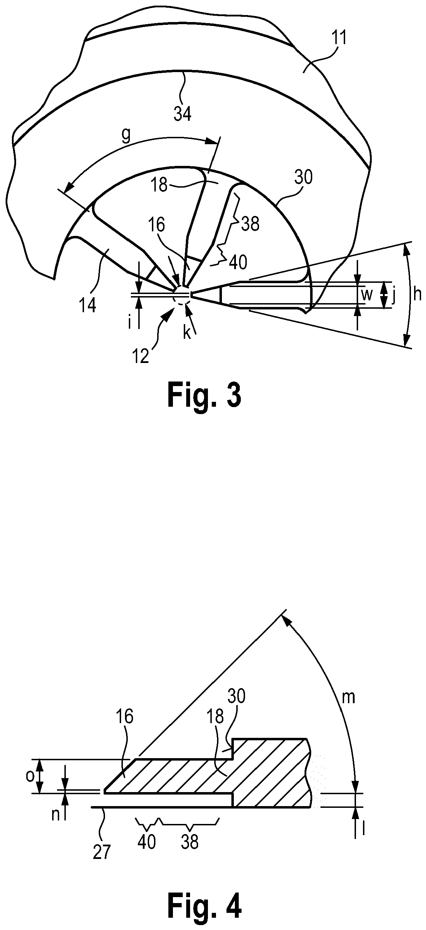

Fig. 3 shows a positioning member 14 of microwell 100 in more detail as a top-view and Fig. 4 shows a positioning member 14 of microwell 100 in more detail as a side-view.

The positioning members 14 can be distributed symmetrically in a circle k having a diameter of 0.3 mm around the sample bed 12 and angled relative to the neighboring positioning members in a third angle g of 72°. In other preferred embodiments, the positioning members 14 can be distributed asymmetrically. As will be clear to the skilled person, in other preferred embodiments, in which another number of symmetrically distributed positioning members 14 is provided or in which the positioning members 14 are distributed asymmetrically, the third angle g can vary. In other preferred embodiments, the circle k can have a diameter of 0.1 mm to 1 mm.

In the embodiment of microwell 100, the positioning members 14 are identical. In other preferred embodiments, at least one positioning member 14 can be different from another positioning member 14.

Each positioning member 14 comprises a first end 16 and a second end 18 and can comprise a spacer 38 and a transition zone 40.

The spacer 38 is located in between the first end 16 and the second end 18.

The shape of the spacer 38 can be a right prism. The base of the prism can be identical to the second end 18 or can abut the second end 18. The base of the prism can be rectangular and can have edge lengths o of 0.5 mm and j of 0.4 mm. A height of the prism can be 1 mm. The prism can be oriented such that a shell side of a short base edge of the rectangular base is horizontal.

In other preferred embodiments, the spacer 38 can have any other shape, as for example the shape of a prism or cylinder, preferably a right prism or cylinder, more preferably a right regular prism or cylinder, said prism or cylinder, right prism or cylinder, or right regular prism or cylinder comprising a base having any shape, preferably the shape of a polygon, preferably a rectangle or triangle, or the shape of a circle or oval, respectively. In all embodiments the shape of the spacer 38 preferably allows a substantially smooth surface omitting small recesses in which dust, cells, bacteria, or other contaminations may accumulate.

The transition zone 40 is located adjacent to the spacer 38 and the first end 16, wherein an outer surface of the transition zone 40 can abut an outer surface of the spacer 38 and the first end 16 each smoothly or obtusely angled. The transition zone 40 can taper from the spacer 38 towards the base of the first end 16.

In other preferred embodiments, the transition zone 40 can have the shape of a prism or cylinder. In such embodiments, the transition zone 40 does not taper.

In the embodiment of microwell 100, the first end 16 can be configured to contact an organoid and can be adjacent to the transition zone 40.

Between the first end 16 and the bottom 26 there can be a distance I of 0.2 mm, which distance I is the smallest distance between the first end 16 and the bottom 26. In other preferred embodiments, the distance I can be from 0.1 mm to 0.5 mm, preferably from 0.15 to 0.25 mm.

The shape of the first end 16 can be a right-angled frustopyramid. A base of the frustopyramid can be rectangular and can have edge lengths w of 0.25 mm and o of 0.5 mm. A height of the frustopyramid can be 0.5 mm. A plumb base point of a tip of the frustopyramid can be in the center of a short edge of the base of the frustopyramid. The pyramidal shape can be truncated parallel to the base of the pyramidal shape. The tip of the frustopyramid can be rectangular and have edge lengths i of 0.05 mm and n of 0.05 mm. The right-angled shell side of the right-angled frustopyramid can be horizontal and face the bottom 26. The two shell sides adjacent to the right-angled shell side can be angled to each other in an angle h of 26.45°. The right-angled shell side and the opposed shell side can be angled to each other in an angle m of 45°.

In other preferred embodiments, the shape of the first end 16 can be any shape, preferably a prism, pyramid, cone, frustopyramid, frustocone, or a convex shape.

In a preferred embodiment, the pyramidal or conical shape of the first end 16 can be truncated.

In another preferred embodiment, in which the shape of the first end 16 is a pyramid, cone, frustopyramid, or frustocone, the plumb base point of the tip of the shape is not located outside the base of the shape, i.e., the shape of the first end 16 is an acute pyramid, cone, frustopyramid, or frustocone.

In a preferred embodiment, the base of the shape of the first end 16 is congruent to the base of the spacer 38. In this embodiment, preferably no transition zone 40 is provided, and the first end 16 is adjacent to the spacer 38.

In another preferred embodiment, the base of the shape of the first end 16 is not congruent to the base of the shape of the spacer 38. In this embodiment, the positioning member 14 preferably comprises a transition zone 40 located in between the spacer 38 and the first end 16, wherein the outer surface of the transition zone 40 abuts the outer surface of the spacer 38 and the first end 16 each smoothly or obtusely angled. The transition zone 40 can have the shape of a prism. Preferably the transition zone 40 tapers from the spacer 38 towards the base of the first end 16.

In embodiments in which the base of the first end 16 is not congruent to the base of the shape of the spacer 38, the base of the shape of the first end 16 is preferably parallel to the base of the shape of the spacer 38 and is further preferably sized as to extend within the base of the shape of the spacer 38.

In the embodiment of microwell 100, the second end 18 is connected to the wall 24, more specifically to the first inner surface 30. The second end 18 can abut partially smoothly (Fig. 3) and/or partially obtusely angled (Fig. 4) to the first inner surface 30. The second end 18 can be identical to the base of the spacer 38 or can abut the base of the spacer 38.

In other preferred embodiments, the second end 18 may be connected to any part of the wall 24, the bottom 26, or both.

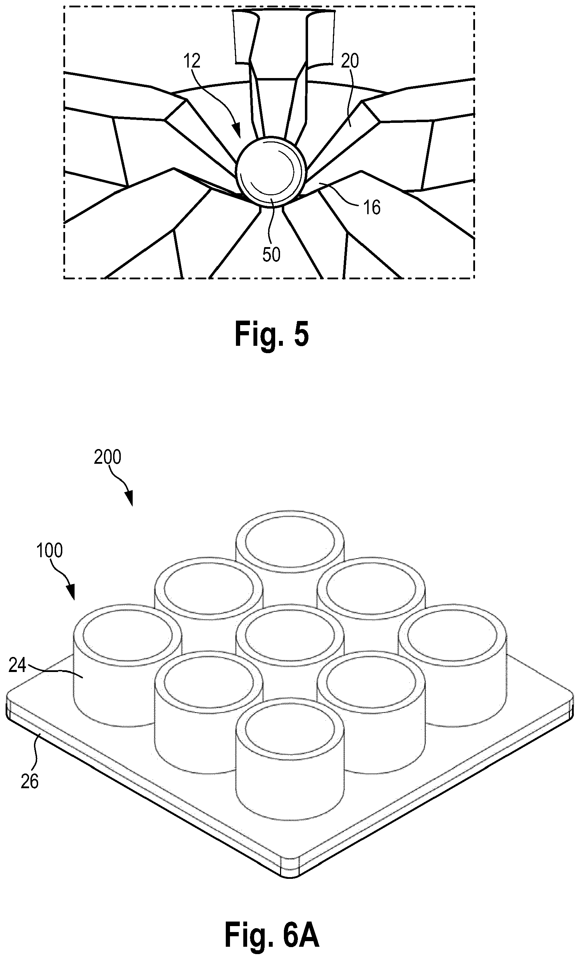

Fig. 5 shows a sample bed 12 with an organoid 50 inserted.

Each first end 16 can comprise a contact-surface 20 and can be configured to contact the organoid 50.

The contact surface 20 can be a shell side of the at least one first end 16 being frustopyramidal. In another preferred embodiment, the contact-surface 20 can be any part of the surface of the first end 16 or the whole surface of the first end 16.

The at least one contact-surface 20 can contact a bottom hemisphere of the organoid 50, i.e., the organoid 50 can lay on the contact-surface 20. In other preferred embodiments, the at least one contact-surface 20 may contact the equator, the bottom hemisphere, and/or the top hemisphere of the organoid 50, preferably the equator and/or the bottom hemisphere.

The at least one contact-surface 20 can be arranged symmetrically and regularly in or as a circle 23. In other embodiments, the at least one contact-surface 20 can be arranged asymmetrically and/or irregularly.

The at least one contact-surface 20 can be flat. In other preferred embodiments, it can be curved (as indicated in Fig. 1), convex, edgy, or flat and the contact-surface 20 can be oriented vertically or substantially vertically, angled, or substantially horizontal.

The organoid 50 can comprise neuronal cells. In other preferred embodiments the organoid 50 can comprise any types of cells. Preferably the organoid 50 comprises cells developing protrusions and/or cells establishing an electric potential difference over its cell membrane, more preferably neuronal and/or muscle cells, most preferably neuronal cells.

The organoid 50 can be spherical. In other preferred embodiments, the organoid 50 can have any shape, preferably a substantially spherical shape, more preferably a spherical shape.

### 2. Device

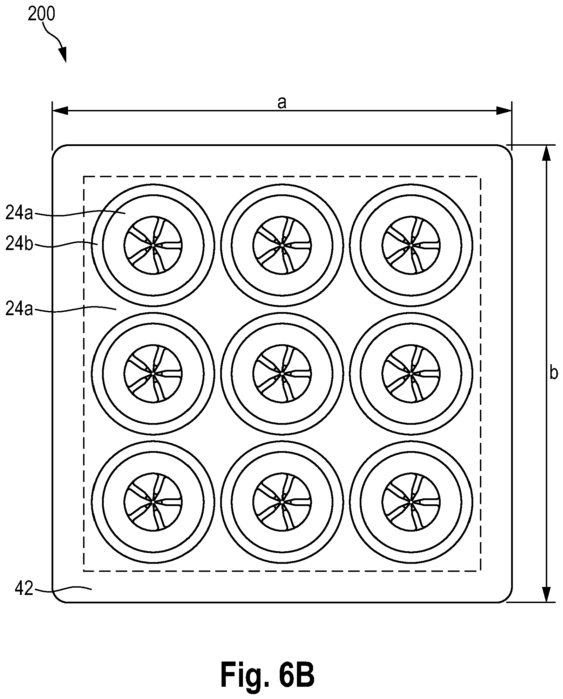

Fig. 6A shows a device 200 for measuring electrical activity of and/or stimulating cells in a three-dimensional network. Fig. 6B shows the device 200 as a top-view. The device 200 comprises a microwell 100.

In the embodiment of device 200, 9 microwells 100 can be provided. In other preferred embodiments up to 5000 microwells 10, 100 may be provided, preferably up to 3456, more preferably 3456, 1536, 384, 96, 48, 24, 12, 11, 10, 9, 8, 7, 6, 5, 4, 3, or 2 microwells, further preferably 96, 10, 9, 8, 7, 6, 5, 4, 3, or 2 microwells, most preferably 9 microwells.

Each microwell 100 can be attached to at least one other microwell 10, 100 at the first section 24a and the bottom 26. In other preferred embodiments, a microwell 10, 100 can be attached to at least one other microwell 10, 100 at any position, for example exclusively at the first section 24a, the bottom 26, or the second section 24b or at a combination thereof.

An attachment can be achieved by any means, as for example gluing, sticking, or screwing. Attachment is preferably achieved by fabricating the first section and/or the bottom 26 and/or the second section 24b of the at least one microwell 10, 100 of device 200 from one piece.

In another preferred embodiment, the wall 24 of the at least one microwell 10, 100 of device 200 are made from a single first piece and the bottom 26 of the respective microwells 10, 100 are made from a single second piece, which first and second piece are attached to each other without being made from one piece.

In other preferred embodiments, a microwell 10, 100 can be a recess in a block of material in which multiple microwells 10, 100, i.e., multiple of such recesses can be provided.

In the embodiment of device 200, the microwells 10, 100 can be distributed regularly in a square array of 3x3 microwells. In other preferred embodiments, the microwells 10, 100 can be distributed irregularly or otherwise regularly according to any geometry.

In the embodiment of device 200, all microwells 10, 100 can be identical. In other preferred embodiments, the microwells 10, 100 can be different.

The device 200 can further comprise a handling member 42.

The handling member 42 can circumferentially and radially extend from the first section 24a and/or bottom 26 of marginal located microwells 10. The handling member 42 can comprise a length a of 32 mm and a width b of 32 mm and can be made from one piece with the first section 24a and/or the bottom 26.

In other preferred embodiments, the handling member 42 can be, dependent on the number and distribution of the microwells 10, of any shape and dimensions. Preferably a length and/or width of the handling member does not exceed 20 cm, more preferably it does not exceed 10 cm.

The handling member 42 can be attached to the first section 24a and/or the bottom 26 of at least one microwell 10, 100, most preferably to all marginal located microwells 10.

An attachment can be achieved by any means, as for example gluing, sticking, or screwing. Attachment is preferably achieved by fabricating the structures to be attached from one piece.

In other preferred embodiments, the handling member 42 can extend to the top or no handling member 42 can be provided.

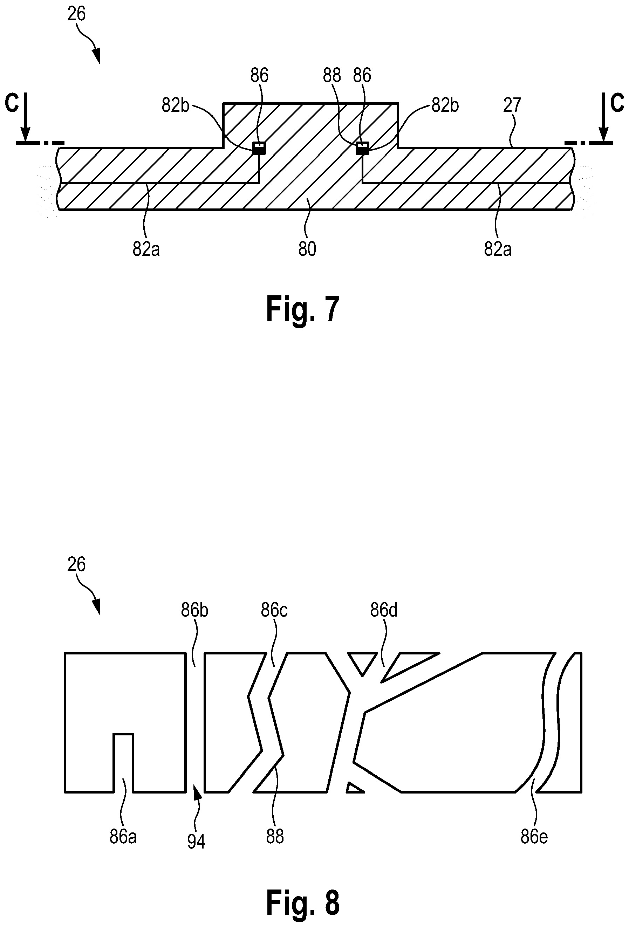

Fig. 7 shows a vertical section through the bottom 26 of a microwell 10, 100. The bottom 26 can comprise a microelectrode array (MEA) 80, at least one microchannel 86, and a first surface 27.

The MEA 80 comprises at least one microelectrode 82. The at least one microelectrode 82 comprises a first part 82a included by the MEA 80 and a second part 82b not included by the MEA 80. The second part 82b is covered by, i.e., within, the at least one microchannel 86.

Fig. 8 shows a horizontal section through the bottom 26 of a microwell 10, 100 with examples of microchannels 86. Fig. 8 can be a section through the plane C indicated in Fig. 7. The at least one microchannel 86 comprises a wall 88 and at least one entrance 94 and is dimensioned as to allow extension of neurites from the first compartment 19a into the microchannel 86 and prevent the entry of the soma of neurons.

In a preferred embodiment, up to 1000 microchannels 86, preferably up to 500, and most preferably up to 100 can be provided.

The wall 88 of the microchannel 86 can be formed at least partially by at least one microelectrode 82. The microelectrode 82 can be positioned at any position along the length or perimeter of the microchannel 86, for example at the bottom of the microchannel 86. There may be more than one microelectrode 82 per microchannel 86. The microelectrode 82 may be positioned on one side of the microchannel 86, along the whole perimeter of the microchannel 86, may be smaller than the length of the microchannel 86, or it may extend over the entire length of a microchannel 86.

The at least one microchannel 86 is in fluid communication with the first compartment 19a via at least one entrance 94.

The microchannel 86 can be for example a dead-end microchannel 86a, a straight pass-through microchannel 86b, a twisted pass-through microchannel 86c, a branched pass-through microchannel 86d, curved or bent pass-through microchannel 86e, or any combination thereof. The microchannel 86a, can comprise a single entrance 94. The microchannel 86 preferably comprises at least two entrances 94.

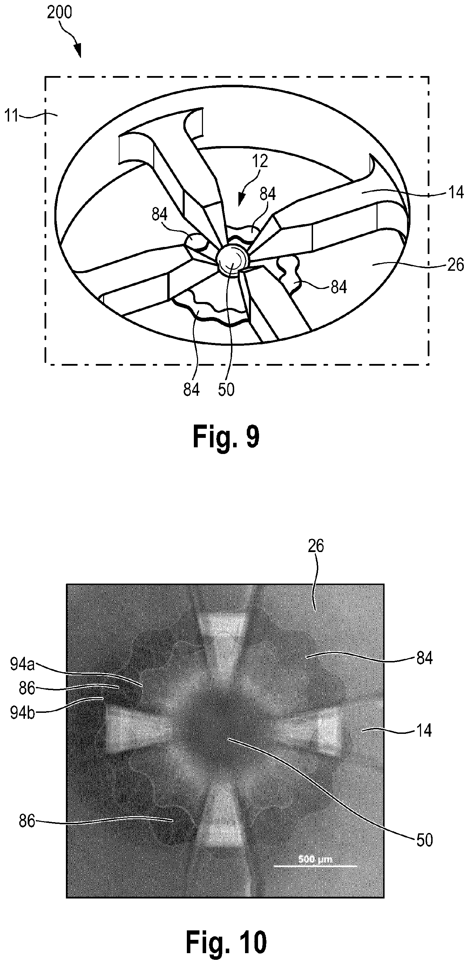

Fig. 9 shows a sketch of a device 200, with an organoid 50 inserted. In this embodiment, the device 200 comprises a microwell 10, 100 comprising a main body 11, a sample bed 12, and 4 positioning members 14.

The at least one microelectrode 82 (not shown) and the at least one microchannel 86 (not shown) can be distributed within a ring-shaped region 84 of the bottom 26, and the sample bed 12 and the organoid 50 can be positioned vertically above a center of the ring-shaped region 84.

In other preferred embodiments, the at least one microelectrode 82 and the at least one microchannel 86 can be distributed within one or more regions of the bottom 26, which region can have any shape, or within the whole bottom 26. The sample bed 12 can be positioned vertically above such region or at any position, which position may or may not be in any defined relationship to such region.

Fig. 10 shows a microscopic photo of a sample bed 12 of this embodiment, with an organoid 50 inserted.

In this embodiment, a plurality of microchannels 86 is distributed within the ring-shaped region 84 of the bottom 26. The microchannels 86 can be pass-through microchannels, being in fluid communication with the first compartment 19a via at least one first entrance 94a directed towards the inner of the ring-shaped region 84 and at least one second entrance 94b directed towards the periphery of the ring-shaped region 84.

In other preferred embodiments, additionally or exclusively one or more other types of microchannels 86, as shown for example in Fig. 8, can be provided.

The organoid 50 shown in Fig. 10 was inserted into the microwell 10 and cultured for a few days. The organoid 50 is still in place after a few days, during which the microwell 10 was handled. Thus, the microwell 10 allows to fixedly position the organoid 50 at a distance from the wall 24 and the bottom 26 of the microwell 10. Fine extensions, i.e., neurites, from the organoid 50 have developed, which partially extend through the microchannels 86. Thus, microwell 10 allows to culture the organoid 50 in a three-dimensional network.

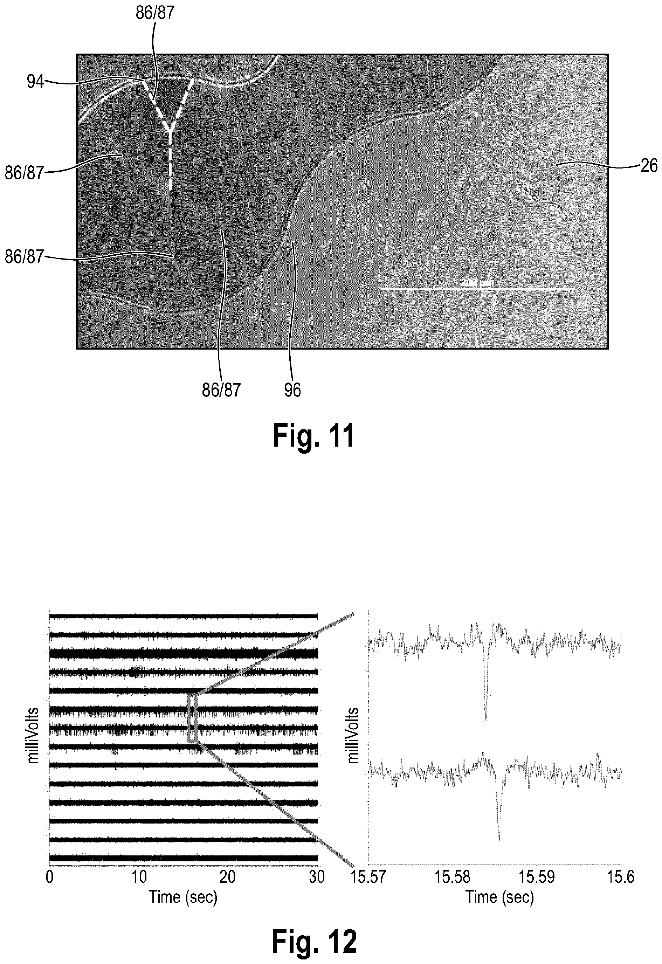

Fig. 11 shows a microscopic photo of a microchannel 86 distributed within a region of the bottom 26.

In this embodiment, at least one of the at least one microchannel 86 comprises 4 Y-shaped segments 87, which Y-shaped segments 87 are connected at the Y's base.

In other preferred embodiments, at least one Y-shaped segment 87, preferably at least 2, most preferably at least 4 are provided. The Y-shaped segments may or may not be connected. Preferably at least 2, further preferably 4, Y-shaped segments are connected at the Y's base.

In this embodiment, the at least one microchannel 86 comprises a width of 7.5 µm, a height of 3 µm, and a length of 250 µm.

In other preferred embodiments, the at least one microchannel 86 can comprise a width and/or a height of up to 20 µm, preferably up to 10 µm and a minimum width and/or height is 1 µm. In a preferred embodiment at least one of the width or height is 3 µm, more preferably 2 µm. In preferred embodiments, the length of the microchannel 86 can be from 50 µm to 500 µm, preferably from 200 µm to 300 µm.

In Fig. 11, neurites 96 extend through the microchannels 86. Thus, the at least one microchannel 86 is dimensioned as to allow extension of neurites from the first compartment 19a into said microchannel 86 and prevent the entry of the soma of neurons.

Fig. 12 shows a measuring obtained at 4 days after plating an organoid 50 comprising neuronal cells in the device 200 comprising the microwell 10. Neurites growing in 3D out of the brain organoid 50 into the hydrogel extend through microchannels 86 located at the bottom 26, which results in a high probability of measuring action potentials propagating along neurites. Thus, the device 200 comprising a bottom 26 comprising a MEA 80 and at least one microchannel 86 allows to directly measure action potentials, i.e., electrical activity of neurites extending from an organoid 50 comprising neuronal cells and cultured in a three-dimensional network with high signal-to-noise ratio. It will be clear to the skilled person how to stimulate the organoid 50, for example by addition of supplements into the liquid culture medium or by using the at least one microelectrode 82 to stimulate the organoid 50 electrically.

### 3. Material

The microwell 10, 100, and the device 200 can be made from any material, preferably from plastic or glass, more preferably from a material selected from the group consisting of: fused silica, borosilicate, allumosilicate, titania-silicate, soda-lime glass, and an elastomer, more preferably from fused silica, borosilicate, allumosilicate, titania-silicate, and soda-lime glass. Most preferably the microwell is made from fused silica.

In preferred embodiments the microwell 10, 100 is at least partially transparent. Preferably, at least a part of the bottom 26 is transparent, more preferably, the whole microwell 10, 100 or device 200 is transparent.

In other preferred embodiments, in which the microwell 10, 100 is at least partially transparent, the microwell 10, 100 or the device 200 can be used in combination with an imaging and/or magnification device, as for example a microscope.

In embodiments in which the microwell 10, 100 or device 200 is made from glass, the microwell 10 or the device preferably is used for measuring electrical activity of and/or stimulating cells electrically.

## Claims

1. A microwell (10; 100) for culturing an organoid (50), the microwell (10; 100) comprising a main body (11), a sample bed (12), and at least one positioning member (14),
the main body (11) comprising a wall (24) and a bottom (26),
the at least one positioning member (14) comprising
- a first end (16), and
- a second end (18) attached to the main body (11),
wherein the sample bed (12) is formed at least partially by the first end (16).

2. The microwell (10; 100) of claim 1, wherein the first end (16) of at least one positioning member (14) is a free end not connected to other first ends (16) and/or the main body (11) other than via the second end (18).

3. The microwell (10; 100) of anyone of claims 1 to 2, wherein the microwell (10; 100) comprises a single positioning member (14) and wherein the first end (16) comprises a ring configured for holding an organoid (50).

4. The microwell (10; 100) of anyone of claims 1 or 2, comprising at least 2 positioning members (14), preferably at least 3, more preferably at least 4, most preferably at least 5.

5. The microwell (10; 100) of anyone of the preceding claims, wherein each of the at least one positioning member (14) extends in a plane, and preferably, the sample bed (12) and each positioning member (14) extend in the same plane.

6. The microwell (10; 100) of anyone of the preceding claims, wherein the at least one positioning member (14) is a straight or bifurcated strut, preferably an I-shaped, Y-shaped or T-shaped strut.

7. The microwell (10; 100) of anyone of the preceding claims, wherein said microwell (10; 100) comprises
- a first compartment (19a) comprising the sample bed (12) and configured to contain a hydrogel, and preferably
- a second compartment (19b) in fluid communication with the first compartment (19a) and configured to contain a liquid culture medium, wherein the second compartment (19b) is located above the first compartment (19a) with respect to gravity.

8. The microwell (10; 100) of anyone of the preceding claims, wherein the organoid (50) comprises neuronal cells.

9. The microwell (10; 100) of anyone of the preceding claims, wherein the microwell (10; 100) is made at least partially from glass or plastic, preferably from a material selected from the group consisting of: fused silica, borosilicate, allumosilicate, titania-silicate, soda-lime glass, and an elastomer.

10. The microwell (10; 100) of anyone of claims 4 or 7 to 9, wherein the first end (16) of the at least two positioning members (14) comprises a fraction of a ring, which fraction of a ring is connected to at least one other fraction of a ring such that a ring configured for holding an organoid (50) is built.

11. A device (200) for measuring electrical activity of and/or stimulating cells in a three-dimensional network, the device (200) comprising at least one microwell (10; 100) according to anyone of the preceding claims, preferably comprising 9 microwells (10; 100).

12. The device (200) of claim 11, wherein the bottom (26) of the at least one microwell (10; 100) comprises a microelectrode array (MEA) (80) comprising at least one microelectrode (82).

13. The device (200) of claim 12, wherein the bottom (26) of the at least one microwell (10; 100) comprises at least one microchannel (86), comprising a wall (88), being in fluid communication with the first compartment (19a), and being dimensioned as to allow extension of neurites from the first compartment (19a) into said microchannel (86) and prevent the entry of the soma of neurons, and wherein the wall (88) of the microchannel (86) is formed at least partially by at least one microelectrode (82).

14. The device (200) of claim 13, wherein the microchannel (86) is branched, and preferably comprises at least one Y-shaped segment (87), wherein preferably at least 2 Y-shaped segments (87), further preferably 4, are connected at the Y's base.

15. The device (200) of anyone of claims 11 to 14 or the microwell (10; 100) of any one of claims 1 to 10, for use in combination with an imaging-device, preferably a microscope.
